# EUROPEAN PATENT SPECIFICATION

(11) **EP 0 464 927 B1**
(45) Date of publication and mention of the grant of the patent: **04.09.1996**
(21) Application number: 91201639.1
(22) Date of filing: 27.06.1991
(51) Int. Cl.: C07D 471/04, A61K 31/435

(54) **3-[(5-Methyl-2-furanyl)methyl]-N-(4-piperidinyl)-3H-imidazo[4,5-b]-pyridin-2-amine 2-hydroxy-1,2,3-propanetricarboxylate**
3-[(5-Methyl-2-furanyl)methyl]-N-(4-piperidinyl)-3H-imidazo[4,5-b]-pyridin-2-amin-2-hydroxy-1,2,3-propantricarboxylat
2-Hydroxy-1,2,3-propane-tricarboxylate de 3-[(5-méthyl-2-furanyl)méthyl]-N-(4-pipéridinyl)-3H-imidazo[4,5-b]-pyridine-2-amine

(30) Priority: 06.07.1990 US 549350
(43) Date of publication of application: 08.01.1992
(73) Proprietor: JANSSEN PHARMACEUTICA N.V., B-2340 Beerse (BE)
(72) Inventor: De Knaep, Alfons Gaston Maria, B-2300 Turnhout (BE); Nelen, Tonny Franciscus Johanna, B-2910 Essen (BE); Janssens, Frans Eduard, B-2820 Bonheiden (BE)
(74) Representative: Wante, Dirk

(56) References cited:
- EP-A- 0 232 937

## Description

In US-4,888,426 there is described and claimed the compound 3-[(5-methyl-2-furanyl)-methyl]-N-(4-piperidinyl)-3H-imidazo[4,5-b]pyridin-2-amine as an intermediate useful in the preparation of antiallergic compounds. The pharmacological properties, in particular the antiallergic activity of said intermediate as well as compositions and the use thereof are described in US-4,835,161. The presently claimed novel salt form has improved physicochemical stability over the prior known base and salt forms.

The present invention is concerned with the novel salt 3-[(5-methyl-2-furanyl)-methyl]-N-(4-piperidinyl)-3H-imidazo[4,5-b]pyridin-2-amine 2-hydroxy-1,2,3-propanetricarboxylate (1:1) which can be represented by the formula

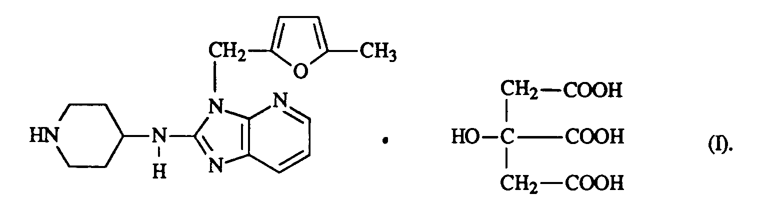

The free base corresponding to the salt of formula (I) is generically known as noberastine and accordingly the novel salt form of formula (I) will hereinafter be referred to as noberastine citrate.

Said free base as well as the dinitrate, the dihydrochloride hemihydrate and the (Z)-2-butenedioate (1:2) salts are described in US-4,835,161. Unfortunately these prior known salts and base all suffer from the disadvantage of not having truly satisfactorily physicochemical stability. Upon storage or formulation of said prior known salts and base, progressive decomposition and concomittantly an increase in the amount and number of impurities is observed. Obviously, this problem is further aggravated under demanding environmental conditions such as light, heat, humidity, acidity, basicity and oxygen.

The free base of noberastine is light-sensitive and degrades significantly when illuminated. Upon storing this yellow compound at 60% or 90% relative humidity, its color changes to dark yellow and water is absorbed. As a consequence the compound partly deliquesces and forms a sticky, solid mass.
The dinitrate and dihydrochloride hemihydrate salts have the drawback of decomposing significantly in aqueous media and consequently are not amenable to standard formulation, especially in substantially liquid compositions. The dihydrochloride hemihydrate salt is hygroscopic and turns from light yellow to dark yellow and eventually to brown.
The (Z)-2-butenedioate (1:2) or dimaleate salt decomposes even at room temperature and the degradation is significantly accelerated by light or enhanced temperatures. A telling observation in this context is the finding that it proves impossible to store samples of this salt for future use as references since invariably at least two impurities are formed.

From the above it follows that none of the prior known salts and base is readily suitable for conventional formulation nor for pharmaceutical use.

Unexpectedly, it has now been found that the above mentioned problems can be circumvented altogether or reduced to negligable proportions by using the citrate salt form of noberastine. This novel salt is not light-sensitive and is far more stable than the prior known salts and base at room temperature, enhanced temperatures, at high relative humidities and in aqueous media.

Noberastine citrate can generally be prepared by dissolving noberastine in a suitable solvent, heating the solution, adding a sufficient quantity of citric acid, cooling the reaction mixture and collecting the crystalline material. The thus obtained noberastine citrate may be further refined by recrystallization.

The free base noberastine as mentioned herein, more particularly when referred to as a starting material for the preparation of the citrate salt, can conveniently be prepared as described in US-4,888,426.

The term suitable solvent as used herein in relation to the preparation of noberastine citrate defines any lower alkanol or ketone solvent in which noberastine is soluble and includes primary, secondary and tertiary alcohols and the corresponding ketones of from 1 to 6 carbon atoms. Suitable lower alkanol solvents include methanol, ethanol, 1-propanol, 2-propanol, 1-butanol, 2-butanol, 2-methyl-1-propanol, 1,1-dimethylethanol and cyclohexanol. Suitable ketone solvents are acetone, butanone, 4-methyl-2-pentanone and cyclohexanone. Mixtures of two or more of the abovementioned solvents may also effectively be employed in the preparation of noberastine citrate, as well as solutions of said solvents or mixtures thereof with water. In particular, the water may comprise up to about 25% to 35% by volume of said solution. Preferably the solvent used is a lower alkanol, particularly methanol or ethanol. Methanol is the most preferred solvent.

The solutions of noberastine in the abovementioned solvents are highly concentrated; typically the amount to volume ratio of noberastine to solvent can range from 0.3 mole/l to 1.2 mole/l, preferably from 0.5 mole/l to 1 mole/l. Said solutions are prepared conventionally by stirring the ingredients at ambient temperature. It is generally appropriate to treat the solution at this point with activated carbon 5% weight by weight of noberastine. Both types of solutions, those comprising activated carbon and also those not, can be stirred for any moment of time up to an hour, preferably for about half an hour and then filtered over diatomaceous earth, which is preferably moistened beforehand with the solvent. In some instances said filtration may prove difficult and can be facilitated considerably by adding an extra amount of diatomaceous earth to the solution. The residue may be washed with small amounts of solvent, generally up to about 25% of the initial volume of solvent used. The combined filtrates are heated at temperatures up to about the reflux temperature of the solution, preferably at temperatures ranging from 45°C to 65°C, particularly temperatures from about 50°C to about 60°C. Citric acid is added portionwise to the heated, concentrated solutions either as a solid or dissolved in a small amount of water. The acid is added at such a rate that the temperature of the solution can easily be maintained constant. The citric acid used may equally well be the anhydrous form or the monohydrate. The molar ratio of citric acid to noberastine can range from about 0.9 to about 2, preferably from about 0.95 to about 1.5 and in particular from about 1.0 to about 1.1. Stirring of the solution is continued at temperatures ranging from 55 to 65°C for any time up to about an hour, preferably for half an hour. The solution is then allowed to cool slowly to ambient temperature. This slow cooling can effectively be accomplished by turning off the heat source and optionally removing said heat source thus facilitating heat exchange with the environment. In case a very large volume to area ratio of the reaction vessel would delay spontaneous cooling too long, the cooling may be accelerated by any cooling means known in the art. After the reaction mixture has cooled the precipitated crystals may optionally be allowed to digest before collection by filtration. Crystal digestion of noberastine citrate may be accomplished by continuing to stir the mixture after room temperature has been reached. Digestion can proceed for any moment of time up to about a day, preferably for about 0 to 4 hours, and in many instances may be left out altogether. The precipitated noberastine citrate is filtered and may be washed with additional solvent, preferably a small amount of cold solvent. The product is then dried by conventional means such as under vacuum and at an enhanced temperature, in particular at about 40°C to about 60°C, preferably at about 50°C. The yield of the thus obtained noberastine citrate ranges from 80% to 95% and is particularly high in methanol, reproducibly giving about 93% to about 94%. The quality of the citrate salt appears not to be influenced by the choice of the solvent.

The thus obtained noberastine citrate may be further refined by recrystallization in a suitable solvent. The term a suitable solvent as used herein in relation to the recrystallization of noberastine citrate defines a lower alkanol solvent as described hereinbefore and also a mixture of such a lower alkanol solvent with water. Particularly mixtures wherein the water comprises up to 30% of the solution by volume may be employed. Particular lower alkanol solvents for recrystallization include methanol, ethanol, 1-propanol and 2-propanol. Solutions of noberastine citrate for recrystallization are highly concentrated, near the saturation point of solute in the solvent and are prepared conventionally from supersaturated solutions of the salt in an aforementioned lower alkanol solvent. Preferably methanol or ethanol is used as lower alkanol solvent and a sufficient amount of water is added, for example, at a later stage of the procedure. Methanol being such a solvent for example, the weight to volume ratio of noberastine citrate to methanol can range from 0.3 g/ml to 0.7 g/ml, particularly from 0.4 g/ml to 0.6 g/ml, preferably from 0.45 to 0.55 g/ml. Said solution in a lower alkanol solvent is stirred and heated up to about the reflux temperature. Water is added dropwise to the heated, heterogenous mixture in order to dissolve the citrate salt completely. Preferably the water is added at such a rate that the temperature of the reaction mixture can be maintained at its reflux temperature. Maintaining a constant temperature of the heated noberastine citrate solution can more easily be accomplished by adding preheated water, preferably having a temperature of about the reflux temperature of the salt solution. After complete addition of the water, the resulting solution is stirred and heated at reflux temperature for any moment of time up to an hour, preferably for about half an hour. The reaction mixture is allowed to cool spontaneously to room temperature and crystallization begins at about 45°C. The crystals are allowed to digest at room temperature for any moment of time up to a day, preferably for about 0 to about 10 hours. The precipitate is filtered off and washed with a small amount of the lower alkanol solvent, typically about 1 ml/g. The crystals are dried in the conventional manner under vacuum and at an enhanced temperature, preferably about 40°C to about 60°C.

In some instances it may be advantageous to incorporate an additional refinement step using activated carbon in the recrystallization procedure. For example, after complete addition of water in the above procedure, activated carbon 5% weight by weight of the citrate salt may be added and further stirred and heated for any time up to an hour, in particular for about half an hour. The heated solution can be filtered over diatomaceous earth, which is preferably moistened beforehand with a preheated mixture of the solvent. The residue may be washed with a similar preheated mixture of the solvent and the combined filtrates are allowed to cool spontaneously further to room temperature. The resulting crystals are then collected and dried as described hereinbefore.

The compound noberastine citrate is an antihistaminic and antiserotoninergic compound useful as an antiallergic agent. Said novel salt, besides the abovementioned physico-chemical stability, further unites in itself the qualities of having good solubility and of being bioequivalent to the (Z)-2-butenedioate (1:2) salt. As the prior known free base and salts, so the citrate salt has a favourable pharmacokinetical profile by uniting a rapid onset and a suitable duration of action.

Noberastine citrate is preferably administered formulated in appropriate pharmaceutical compositions such as, for example, oral solid preparations, e.g. pills, tablets, powders and capsules; oral liquid forms, e.g. solutions, suspensions, syrups and elixirs. Said and similar art-known compositions can be prepared by intimately mixing the active ingredient noberastine citrate with one or more suitable carriers and/or adjuvants and converting the thus obtained mixture in a form suitable for administration.

For ease of administration and uniformity of dosage, it is particularly advantageous to formulate the aforementioned pharmaceutical compositions in dosage unit form. Examples of such dosage unit forms are tablets, capsules, pills, powder packets, wafers, injectable solutions, suspensions, teaspoonfuls, tablespoonfuls and segregated multiples thereof.

An appropriate daily dose of noberastine citrate is contemplated to range between about 0.01 mg/kg to about 10 mg/kg body weight, in particular between 0.05 mg/kg and 5 mg/kg body weight, preferably between 0.1 and 2 mg/kg body weight.

In a further aspect of the invention there is provided the use of noberastine citrate for the manufacture of a medicament for treating individuals suffering from allergic diseases or disorders such as, for example, allergic rhinitis, allergic conjuctivitis, chronic urticaria and allergic asthma.

Yet another aspect of the invention relates to a method of treating individuals suffering from allergic diseases or disorders, by administering an effective antiallergic amount of noberastine citrate to said individuals.

In the following examples, unless otherwise stated, all parts are by weight.

### Experimental part

### Example 1

To a mixture of 60.73 parts of 3-[(5-methyl-2-furanyl)methyl]-N-(4-piperidinyl)-3H-imidazo[4,5-b]pyridin-2-amine (noberastine) in 195 ml of methanol there were added 3.3 parts of activated charcoal (Norit A Supra®) and 9.1 parts of diatomaceous earth. The reaction mixture was stirred for 1/2 hour at 20°C and filtered over diatomaceous earth, which was then rinsed with 40 ml of methanol. The combined filtrates were heated at 50°C and 41 parts of citric acid monohydrate were added. The mixture was stirred for 1/2 hour at about 60°C to 65°C and was then allowed to cool spontaneously to room temperature. The mixture was stirred overnight and filtered off. The precipitate was washed with 80 ml of methanol and dried in vacuo at 50°C, yielding 91.2 parts (92.9%) of 3-[(5-methyl-2-furanyl)methyl]-N-(4-piperidinyl)-3H-imidazo[4,5-b]-pyridin-2-amine 2-hydroxy-1,2,3-propanetricarboxylate (1:1) (noberastine citrate); mp. 192.0°C.

### Example 2

A stirred mixture of 40 parts of noberastine citrate (as prepared in Example 1) in 80 ml of methanol was heated at reflux temperature. About 25 ml of water was added and stirring at reflux temperature was continued for 1/2 hour. The solution was allowed to cool spontaneously to room temperature (20°C) and was further stirred overnight. The crystalline precipitate was filtered off, washed with methanol and dried in vacuo at 50°C, yielding 37.4 parts (94%) of noberastine citrate.

### Example 3

A stirred mixture of 40 parts of noberastine citrate (as prepared in Example 1) in 80 ml of methanol was heated at reflux temperature. About 25 ml of water and 2 parts of activated charcoal were added and stirring at reflux temperature was continued for 1/2 hour. The solution was filtered over diatomaceous earth, which was rinsed with a preheated mixture of 16 ml of methanol and 5 ml of water. The combined filtrates were allowed to cool spontaneously to room temperature and were further stirred overnight. The crystalline product was collected, washed with methanol and dried in vacuo at 50°C, yielding 34.7 parts (87%) of noberastine citrate; mp. 191.8°C.

### Example 4 : Light stability

150 mg portions of noberastine base, noberastine dimaleate and noberastine citrate were weighed into glass containers. The containers were irradiated for 7 days in a light cabinet by 16 cool white fluorescent tubes (Sylvania F 20 T 12/D Daylight - 6500 K). The light intensity was 17000 lux and the mean temperature in the cabinet was approximately 40°C. After 3 and 7 days of illumination a 50 mg portion of each sample was dissolved in 10 ml of methanol-water (2:3, v/v) and was analyzed by HPLC.
column: 12.5 cm Superspher RP Select-B (4µm)
flow rate: 1.5 ml/min.
elution solvents: A : 0.75% ammonium acetate in water
B : acetonitrile
C : tetrahydrofuran
elution mode : linear gradient

| time (min.) | 0 | 4 | 12 | 15 |
|---|---|---|---|---|
| %A | 91 | 85 | 60 | 30 |
| %B | 9 | 15 | 20 | 45 |
| %C | 0 | 0 | 20 | 25 |

detection : U.V. (220 nm)

The following table shows the relative retention time (RRT; versus noberastine) and concentration (%) of each decomposition product detected at a concentration ≧ 0.1% in the analyzed samples.

| noberastine | RRT | 0.25 | 0.61 | 0.72 | 1.23 | 1.26 | 1.43 | 1.45 | 1.59 | 1.70 | 1.73 |
|---|---|---|---|---|---|---|---|---|---|---|---|
| base | 3d | 0.2 | 0.2 | | 0.3 | 0.1 | 0.2 | 0.2 | | | 0.1 |
| | 7d | 0.3 | 0.3 | | 0.4 | 0.2 | 0.3 | 0.2 | 0.2 | 0.1 | 0.1 |
| dimaleate | 3d | | 0.8 | 0.5 | | | | | | | |
| | 7d | | 1.2 | 0.8 | | | | | | | |
| citrate | 3d | | | | | | | | | | |
| | 7d | | | | | | | | | | |

Whereas the base and the dimaleate of noberastine decomposed significantly under irradiation by daylight, noberastine citrate remained stable when stored at the investigated conditions and no discoloration nor degradation could be observed.

### Composition Examples

The following formulations exemplify typical pharmaceutical compositions in dosage unit form suitable for systemic or topical administration to warm-blooded animals in accordance with the present invention.

"Active ingredient" (A.I.) as used throughout these examples relates to noberastine citrate.

### Example 5 : Oral drops

500 g of the A.I. is dissolved in 0.5 l of 2-hydroxypropanoic acid and 1.5 l of the polyethylene glycol at 60∼80°C. After cooling to 30~40°C there are added 35 l of polyethylene glycol and the mixture is stirred well. Then there is added a solution of 1750 g of sodium saccharin in 2.5 l of purified water and while stirring there are added 2.5 l of cocoa flavor and polyethylene glycol q.s. to a volume of 50 l, providing an oral drop solution comprising 10 mg/ml of the A.I. The resulting solution is filled into suitable containers.

### Example 6 : Oral solutions

9 g of methyl 4-hydroxybenzoate and 1 g of propyl 4-hydroxybenzoate are dissolved in 4 l of boiling purified water. In 3 l of this solution are dissolved first 10 g of 2,3-dihydroxybutanedioic acid and thereafter 20 g of the A.I. The latter solution is combined with the remaining part of the former solution and 12 l of 1,2,3-propanetriol and 3 l of sorbitol 70% solution are added thereto. 40 g of sodium saccharin are dissolved in 0.5 l of water and 2 ml of raspberry and 2 ml of gooseberry essence are added. The latter solution is combined with the former, water is added q.s. to a volume of 20 l providing an oral solution comprising 5 mg of the A.I. per teaspoonful (5 ml). The resulting solution is filled in suitable containers.

### Example 7 : Capsules

20 g of the A.I., 6 g sodium lauryl sulfate, 56 g starch, 56 g lactose, 0.8 g colloidal silicon dioxide, and 1.2 g magnesium stearate are vigorously stirred together. The resulting mixture is subsequently filled into 1000 suitable hardened gelatin capsules, each comprising 20 mg of the A.I..

### Example 8 : Film-coated tablets

### Preparation of tablet core

A mixture of 100 g of the A.I., 570 g lactose and 200 g starch is mixed well and thereafter humidified with a solution of 5 g sodium dodecyl sulfate and 10 g polyvinylpyrrolidone (Kollidon-K 90®) in about 200 ml of water. The wet powder mixture is sieved, dried and sieved again. Then there are added 100 g microcrystalline cellulose (Avicel®) and 15 g hydrogenated vegetable oil (Sterotex ®). The whole is mixed well and compressed into tablets, giving 10.000 tablets, each comprising 10 mg of the active ingredient.

### Coating

To a solution of 10 g methyl cellulose (Methocel 60 HG®) in 75 ml of denaturated ethanol there is added a solution of 5 g of ethyl cellulose (Ethocel 22 cps ®) in 150 ml of dichloromethane. Then there are added 75 ml of dichloromethane and 2.5 ml 1,2,3-propanetriol. 10 g of polyethylene glycol is molten and dissolved in 75 ml of dichloromethane. The latter solution is added to the former and then there are added 2.5 g of magnesium octadecanoate, 5 g of polyvinylpyrrolidone and 30 ml of concentrated colour suspension (Opaspray K-1-2109®) and the whole is homogenated. The tablet cores are coated with the thus obtained mixture in a coating apparatus.

## Claims (Claims for the following Contracting State(s): AT, BE, CH, DE, DK, FR, GB, IT, LI, LU, NL, SE)

1. The compound 3-[(5-methyl-2-furanyl)methyl]-N-(4-piperidinyl)-3H-imidazo-[4,5-b]pyridin-2-amine 2-hydroxy-1,2,3-propanetricarboxylate (1:1).

2. A pharmaceutical composition comprising an inert carrier and as an active ingredient an effective antiallergic amount of the compound as claimed in claim 1.

3. A process for preparing a pharmaceutical composition according to claim 2 characterized in that a therapeutically effective amount of the compound as claimed in claim 1 is intimately mixed with a pharmaceutically acceptable carrier.

4. A process for preparing the compound as claimed in claim 1 which comprises dissolving noberastine in a solvent, heating the solution up to about the reflux temperature, adding a sufficient quantity of citric acid, cooling the reaction mixture and collecting the crystalline product; and, if desired, further refining the thus obtained product by recrystallization.

5. The compound as claimed in claim 1 for use as a medicament.

## Claims (Claims for the following Contracting State(s): GR, ES)

1. A process for preparing the compound 3-[(5-methyl-2-furanyl)methyl]-N-(4-piperidinyl)-3H-imidazo-[4,5-b]pyridin-2-amine 2-hydroxy-1,2,3-propanetricarboxylate (1:1) which comprises dissolving noberastine in a solvent, heating the solution up to about the reflux temperature, adding a sufficient quantity of citric acid, cooling the reaction mixture and collecting the crystalline product; and, if desired, further refining the thus obtained product by recrystallization.

2. A process for preparing a pharmaceutical composition according to claim 1 characterized in that a therapeutically effective amount of the compound as claimed in claim 1 is intimately mixed with a pharmaceutically acceptable carrier.

## Patentansprüche (Patentansprüche für folgende(n) Vertragsstaat(en): AT, BE, CH, DE, DK, FR, GB, IT, LI, LU, NL, SE)

1. Die Verbindung 3-[(5-Methyl-2-furanyl)methyl]-N-(4-piperidinyl)-3H-imidazo[4,5-b]pyridin-2-amin-2-hydroxy-1,2,3-propantricarboxylat (1:1).

2. Pharmazeutische Zusammensetzung, enthaltend einen inerten Träger und eine antiallergisch wirksame Menge der Verbindung gemäß Anspruch 1 als Wirkstoff.

3. Verfahren zur Herstellung einer pharmazeutischen Zusammensetzung gemäß Anspruch 2, dadurch gekennzeichnet, daß man eine therapeutisch wirksame Menge der Verbindung gemäß Anspruch 1 innig mit einem pharmazeutisch unbedenklichen Träger vermischt.

4. Verfahren zur Herstellung der Verbindung gemäß Anspruch 1, bei dem man Noberastin in einem Lösungsmittel auflöst, die Lösung bis etwa zur Rückflußtemperatur erwärmt, eine ausreichende Menge Zitronensäure zugibt, die Reaktionsmischung abkühlt und das kristalline Produkt isoliert sowie gegebenenfalls das so erhaltene Produkt durch Umkristallisieren weiter reinigt.

5. Verbindung gemäß Anspruch 1 zur Verwendung als Arzneimittel.

## Patentansprüche (Patentansprüche für folgende(n) Vertragsstaat(en): GR, ES)

1. Verfahren zur Herstellung der Verbindung 3-[(5-Methyl-2-furanyl)methyl]-N-(4-piperidinyl)-3H-imidazo[4,5-b]-pyridin-2-amin-2-hydroxy-1,2,3-propantricarboxylat (1:1), bei dem man Noberastin in einem Lösungsmittel auflöst, die Lösung bis etwa zur Rückflußtemperatur erwärmt, eine ausreichende Menge Zitronensäure zugibt, die Reaktionmischung abkühlt und das kristalline Produkt isoliert sowie gegebenenfalls das so erhaltene Produkt durch Umkristallisieren weiter reinigt.

2. Verfahren zur Herstellung einer pharmazeutischen Zusammensetzung enthaltend einen inerten Träger und eine antiallergisch wirksame Menge der Verbindung gemäß Anspruch 1 als wirkstoff, dadurch gekennzeichnet, daß man eine therapeutisch wirksame Menge der Verbindung gemäß Anspruch 1 innig mit einem pharmazeutisch unbedenklichen Träger vermischt.

## Revendications (Revendications pour l'(les) Etat(s) contractant(s) suivant(s): AT, BE, CH, DE, DK, FR, GB, IT, LI, LU, NL, SE)

1. Le composé 2-hydroxy-1,2,3-propanetricarboxylate de 3-[(5-méthyl-2-furanyl)méthyl]-N-(4-pipéridinyl)-3H-imidazo[4,5-b]pyridine-2-amine (1:1).

2. Une composition pharmaceutique comprenant un véhicule inerte et, comme substance active, une quantité antiallergique efficace du composé selon la revendication 1.

3. Un procédé de préparation d'une composition pharmaceutique selon la revendication 2, caractérisé en ce que l'on mélange intimement une quantité thérapeutiquement efficace du composé selon la revendication 1 avec un véhicule pharmaceutiquement acceptable.

4. Un procédé de préparation du composé selon la revendication 1, qui comprend les étapes qui consistent à dissoudre la nobérastine dans un solvant, chauffer la solution aux environs de la température de reflux, ajouter une quantité suffisante d'acide citrique, refroidir le mélange réactionnel et recueillir le produit cristallin; et, le cas échéant, raffiner davantage par recristallisation le produit ainsi obtenu.

5. Le composé selon la revendication 1, à utiliser comme médicament.

## Revendications (Revendications pour l'(les) Etat(s) contractant(s) suivant(s): GR, ES)

1. Un procédé de préparation du composé 2-hydroxy-1,2,3-propanetricarboxylate de 3-[(5-méthyl-2-furanyl)méthyl]-N-(4-pipéridinyl)-3H-imidazo[4,5-b]pyridine-2-amine (1:1), qui comprend les étapes qui consistent à dissoudre la nobérastine dans un solvent, chauffer la solution aux environs de la température de reflux, ajouter une quantité suffisante d'acide citrique, refroidir le mélange réactionnel et recueillir le produit cristallin; et, le cas échéant, raffiner davantage par recristallisation la produit ainsi obtenu.

2. Un procédé de préparation d'une composition pharmaceutique comprenant un véhicule inerte et, comme substance active, unde quantité antiallergique efficace du composé selon la revendication 1, caractérisé en ce que l'on mélange intimement une quantité thérapeutiqement efficace du composé selon la revendication 1 avec un véhicule pharmaceutiquement acceptable.
